# EUROPEAN PATENT APPLICATION

(11) **EP 4 458 965 A1**
(43) Date of publication of application: **06.11.2024**
(21) Application number: 22914844.0
(22) Date of filing: 28.12.2022
(51) Int. Cl.: C12N 15/10, B01J 20/10, H01F 1/42

(54) **MAGNETIC BEAD, AND PREPARATION METHOD THEREFOR AND USE THEREOF IN NUCLEIC ACID EXTRACTION**

(30) Priority: 28.12.2021 CN 202111623907
(71) Applicant: Nanjing GenScript Biotech Co., Ltd., Nanjing, Jiangsu 211122 (CN)
(72) Inventor: SUN, Liwei, Nanjing, Jiangsu 211122 (CN); LIU, Juncheng, Nanjing, Jiangsu 211122 (CN); JIN, Peng, Nanjing, Jiangsu 211122 (CN); LIU, Siwen, Nanjing, Jiangsu 211122 (CN); ZHOU, Liang, Nanjing, Jiangsu 211122 (CN); ZHANG, Suya, Nanjing, Jiangsu 211122 (CN)
(74) Representative: Laufhütte, Dieter
(86) International application number: PCT/CN2022/142572
(87) International publication number: WO 2023/125592

(57) **Abstract**

A magnetic bead, and a preparation method therefor and the use thereof in nucleic acid extraction. The preparation method for a magnetic bead comprises: preparing a magnetic core by means of a water-soluble salt of Fe²⁺ and Fe³⁺; and subjecting the magnetic core and a silanide to a modification reaction to obtain the magnetic bead, wherein the molar ratio of the addition amount of the used silanide to the total amount of Fe²⁺ and Fe³⁺ is greater than or equal to 0.5 : 1, and the particle size range of the magnetic bead is 0.1-50 µm.

## Description

### Cross-Reference:

The present application claims priority to Chinese Application No. 202111623907.X, field on December 28, 2021, which is incorporated herein by reference in its entirety.

### Technical Field

The present invention relates to the technical field of nucleic acid extraction, in particular to a magnetic bead, and a preparation method therefor and the use thereof in nucleic acid extraction.

### Background Art

Following the advancement of molecular biology, isolation and purification of target nucleic acids from cells or tissues has become a necessary means for technical research. It is a critical step in isolation and purification to achieve cell membrane rupture through mechanical means, chemical lysis or enzymatic digestion. In proceeding this way, however, the target nucleic acid is inevitably contaminated with substantial quantities of additional biological molecules in the lysate. Therefore, it is necessary to remove as many non-target impurities as possible prior to further isolation of target nucleic acids. Currently, to address the above-mentioned problem, two main methods are commonly used: centrifugation and filtration. However, these two methods greatly limit sample throughput and purification efficiency and cannot allow for a fully automatized nucleic acid purification process.

A number of different materials and methods have been developed for the isolation of nucleic acid species from lysates. Many methods are mainly based on silica materials, such as controlled-pore glass, a filter embedded with silica particles, silica gel particles, silica resins, glass fibres, etc. These silica-based solid phase isolation systems can reversibly bind to nucleic acid species in the presence of chaotropic agents, and then the additional contaminating impurities can be separated from the immobilized nucleic acid species by external forces (such as centrifugation and vacuum filtration). Finally, the nucleic acid species are eluted with water or elution buffers from the silica solid-phase material. Although the above-mentioned methods optimize the nucleic acid molecule purification process to a certain extent, repeated centrifugation or filtration is required to complete the entire isolation and purification step, which limits the practicality of such methods in automated systems.

Magnetic microspheres have been used in automated nucleic acid purification methods. However, in these methods, there are often a large number of additional contaminants in cell lysates, and impurities, such as substantial quantities of proteins or cell and tissue debris, can be entangled with or adsorbed to nucleic acids. Consequently, when the nucleic acids bind to magnetic beads, these impurities will be adsorbed on the surface of the magnetic beads and cannot be well removed in the subsequent washing process.

Magnetic beads, which are currently used for nucleic acid extraction, usually contain silicon hydroxyl modification. The methods for preparing these magnetic beads are as disclosed in CN 106215821B (the method comprises: subjecting FeCl₃·6H₂O and sodium acetate to a solvothermal reaction to produce magnetic microspheres, and performing a modification reaction by adding ethyl orthosilicate, wherein the molar ratio of ferroferric oxide to ethyl orthosilicate is 10: 3) and CN 112246196B (the method comprises: reacting 10 g of FeCl₂·4H₂O with 15 g of FeCl₃ 6H₂O to obtain a magnetic sphere seed, modifying the magnetic sphere seed with polyvinylpyrrolidone, and then preparing a functionalization layer with 3 ml of ethyl orthosilicate). During the purification of supercoiled structure-containing nucleic acids, such as plasmids, the proportion of native supercoiled plasmids is often reduced after the conventional steps of magnetic bead adsorption, impurity removal, and binding nucleic acid purification, resulting in compromised plasmid transcription function. Moreover, the loading capacity of commercial magnetic beads is not satisfactory.

In view of the above-mentioned problems, materials and methods for removing from cells or animal tissues as many contaminants (such as proteins and cell debris) as possible to obtain high-purity nucleic acids are crucial for the automated purification of nucleic acids.

### Summary of the Invention

A first objective of the present invention is to provide a preparation method for a magnetic bead, comprising:
preparing a magnetic core by means of a water-soluble salt of Fe²⁺ and Fe³⁺; and
subjecting the magnetic core and a silanide to a modification reaction to obtain the magnetic bead,
wherein the molar ratio of the addition amount of the used silanide to the total amount of Fe²⁺ and Fe³⁺ is (0.5 : 1)-(4.0 : 1), and the particle size range of the magnetic bead is 0.1 µm-50 µm.

A second objective of the present invention is to provide a magnetic bead prepared by the method as described above.

A third objective of the present invention is to provide the use of the magnetic bead as described above in nucleic acid extraction.

A fourth objective of the present invention is to provide a method for extracting a nucleic acid, comprising:
providing a biological sample containing a free nucleic acid and non-nucleic acid impurities, an impurity-removing magnetic bead and a binding magnetic bead, wherein the binding magnetic bead is the magnetic bead as described above, and the impurity-removing magnetic bead and the binding magnetic bead are the same or different magnetic bead(s) except that the modification groups on the surface of the two magnetic beads and/or the modification amount of the modification group on the surface of the two magnetic beads are/is different;
adsorbing the non-nucleic acid impurities to the impurity-removing magnetic bead in a solution system that does not facilitate the adsorption of the nucleic acid to the modification group, and removing the impurity-removing magnetic bead; and
adsorbing the nucleic acid to the binding magnetic bead in a solution system that facilitates the adsorption of the nucleic acid to the modification group, and eluting and harvesting the nucleic acid from the binding magnetic bead.

A fifth objective of the present invention is to provide a nucleic acid extraction kit comprising the magnetic bead as described above.

The present invention further relates to a method for extracting a nucleic acid, comprising:
providing a biological sample containing a free nucleic acid and non-nucleic acid impurities; adsorbing the non-nucleic acid impurities to an impurity-removing magnetic bead; and
removing the impurity-removing magnetic bead by adding a magnetic field to obtain a solution comprising the nucleic acid,
wherein the impurity-removing magnetic bead comprises silicon hydroxyl, carboxyl or a modification group having ion exchange properties.

In the preparation method for a magnetic bead provided by the present invention, an excessive amount of a silanide relative to iron element is added during the modification process, and the magnetic bead obtained thereby, when used in nucleic acid extraction, can not only achieve a higher nucleic acid extraction yield, but also effectively increase extracted nucleic acids (such as plasmid DNA) in the supercoiled form.

By means of the method for extracting a nucleic acid provided by the present invention, nucleic acid binding and purification can be achieved with high efficiency, resulting in high-quality nucleic acids. Moreover, the method also readily enables the fully automated nucleic acid purification of various samples. The nucleic acid isolated according to the method can be used in enzyme digestion, quantitative fluorescence PCR, sequencing, transfection, etc.

### Brief Description of the Drawings

To describe the technical solutions in the specific embodiments of the present invention or in the prior art more clearly, the accompanying drawings required for describing the specific embodiments or the prior art will be briefly described below; obviously, the accompanying drawings described below are some of the embodiments of the present invention; and for those of ordinary skill in the art, additional drawings can be obtained according to these accompanying drawings without involving any inventive effort.

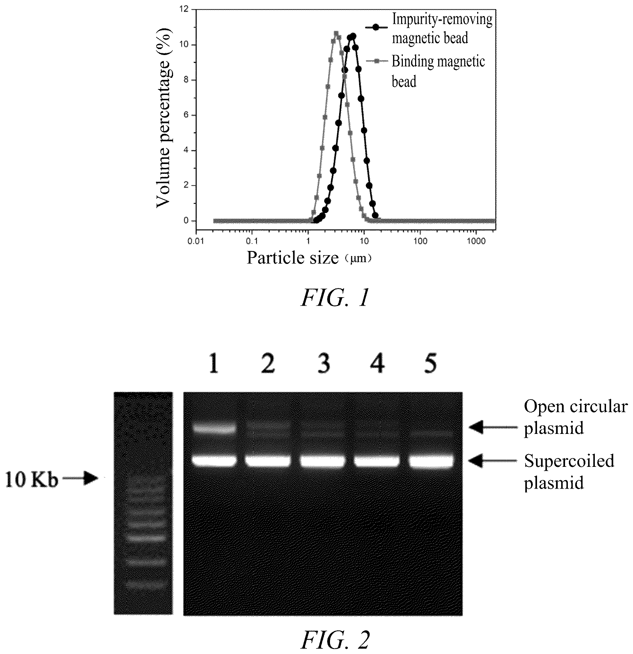

FIG. 1 shows the particle size distribution of the impurity-removing magnetic bead and the binding magnetic bead provided by one example of the present invention.
FIG. 2 shows the effect of magnetic beads prepared with different amounts of ethyl orthosilicate on the supercoiled conformation of purified plasmids in one example of the present invention, wherein lanes 1-4 represent the plasmid samples purified with magnetic beads prepared with 50 mL, 75 mL, 100 mL and 125 mL of ethyl orthosilicate, respectively, and lane 5 represents the plasmid sample purified with a commercial plasmid extraction kit.

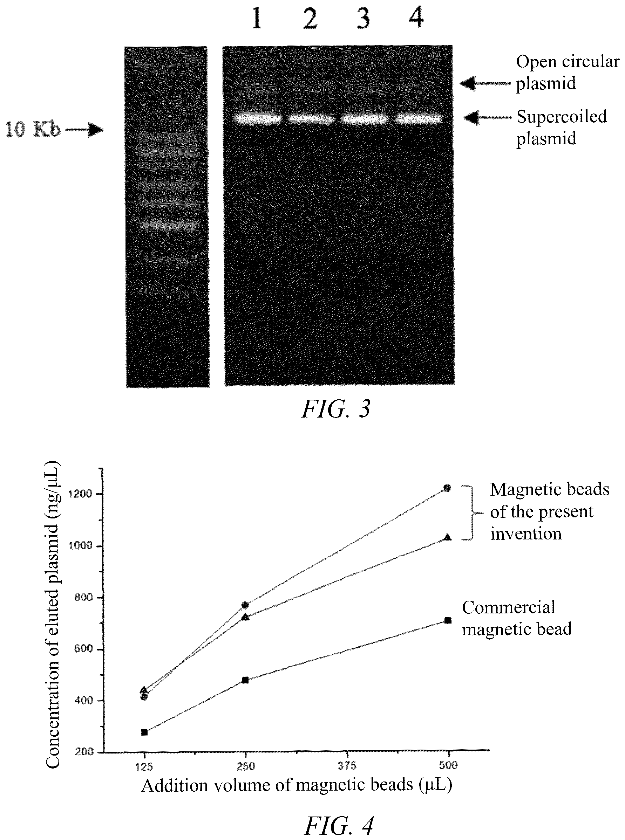

FIG. 3 shows the effect of excessive ethyl orthosilicate added during the binding magnetic bead preparation process on the supercoiled conformation of purified plasmids in one example of the present invention, wherein lanes 1-3 represent the plasmid samples purified with three binding magnetic beads prepared with 150 mL, 200 mL and 250 mL of ethyl orthosilicate, respectively, and lane 4 represents the plasmid sample purified with a commercial plasmid extraction kit.
FIG. 4 shows the effect of adsorption capacity of the binding magnetic beads to plasmid DNA in one example of the present invention.

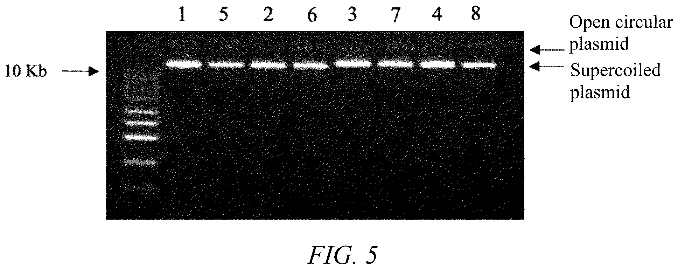

FIG. 5 shows a gel electrophoresis diagram of plasmid DNA extracted from different strains by the method for extracting a nucleic acid in one example of the present invention, wherein lanes 1-4 represent the plasmid samples from strains 1#-4# purified by the method for extracting a nucleic acid of the present invention, respectively, and lanes 5-8 represent the plasmid samples from strains 1#-4# purified with a commercial plasmid extraction kit.

### Detailed Description of Embodiments

Reference now will be made in detail to the embodiments of the invention, one or more examples of which are set forth below. Each example is provided as an explanation rather than limiting the present invention. Indeed, it would have been obvious to a person skilled in the art that various modifications and variations may be made to the present invention without departing from the scopes or spirits of the present invention. For instance, features illustrated or described as part of one embodiment, can be used on another embodiment to yield a still further embodiment.

Unless otherwise stated, all terms (including technical and scientific terms) used to disclose the present invention have the same meaning as commonly understood by those of ordinary skill in the art to which the present invention belongs. With further guidance, ensuing definitions are used to better understand the teachings of the present invention. Herein, the terms used in the description of the present invention are merely for the purpose of describing specific embodiments, but are not intended to limit the present invention.

The terms "and/or" and "or/and" used herein are selected to encompass any one of two or more associated items listed therein, as well as any and all combinations of the associated items listed therein, wherein the combinations include combinations of any two of the associated items listed therein, any more of the associated items listed therein, or all of the associated items listed therein. It should be noted that when at least three items are connected by a combination of at least two conjunctions selected from "and/or" and "or/and", it should be understood that in the present application, the technical solutions definitely include the technical solution in which items are all connected by "logic AND" and also definitely include the technical solutions in which items are all connected by "logic OR". For example, "A and/or B" includes the three parallel solutions of A, B and A+B. As another example, the technical solution of "A, and/or, B, and/or, C, and/or, D" includes any one of A, B, C, and D (i.e., the technical solutions in which items are all connected by "logic OR"), also includes any and all combinations of A, B, C, and D, that is, combinations of any two or any three of A, B, C, and D, and further includes the combination of all the four items A, B, C, and D (i.e., the technical solution in which items are all connected by "logic AND").

In the present invention, the "magnetic bead" may comprise iron element, or a complex formed by iron element with other metals or non-metals, etc. Other metals are, for example, alnico, etc.; and the complex formed by metals with non-metals is, for example, a neodymium-iron-boron rubber magnetic composite material. The magnetic substance is preferably prepared from a water-soluble salt of Fe²⁺ and Fe³⁺, and the water-soluble salt may be one of or a mixture of more of chlorides, sulphates, nitrates and hydrates thereof that can be hydrolysed to obtain Fe²⁺ and Fe³⁺.

In the present invention, the term "magnetic bead" may be spheres, ellipsoids, cubes, polyhedrons or irregular shapes.

In the present invention, the silanide refers to a silicon-substituted analogue of an alkane or an alkane containing a carboxylic acid group, oxy group, keto group and/or ester group. The silanide is composed of a main chain formed by silicon atoms linked together, and hydrogen atoms, oxygen atoms, carbon atoms, etc. linked to the main chain via covalent bonds.

The terms "contain", "comprise", and "include" used herein are synonymous, inclusive or open-ended, and do not exclude additional, unrecited members, elements, or method steps.

Numerical ranges expressed by endpoints in the present invention include all numbers and fractions included within the range, as well as the recited endpoints.

The present invention relates to a concentration value, and the fluctuations of the value are within a certain range. For example, it can fluctuate within the corresponding precision range. For example, with regard to the value 2%, fluctuations within the range of ± 0.1% may be permitted. For larger values or values that do not require way too fine control, the meaning is also allowed to include larger fluctuations. For example, with regard to the value 100 mM, fluctuations within the range of ± 1%, ± 2%, ± 5%, etc. may be permitted. The present invention relates to a molecular weight and the meaning of its value is allowed to include fluctuations with the range of ± 10%.

In the present invention, expressions involving terms such as "a plurality of" and "multiple" refer to a quantity greater than or equal to 2, unless otherwise specified.

In the present invention, the technical features described in an open-ended manner include both a closed technical solution consisting of the listed features and an open-ended technical solution comprising the listed features.

In the present invention, the expressions "preferably", "preferentially", "more preferably", and "appropriately" are solely used for describing better embodiments or examples, and it should be understood that the scope of the present invention is not intended to be limited. In the present invention, the expressions "optionally", "optional" and "alternative" mean that the subject modified by the expressions are dispensable, that is, the expressions mean that the parallel technical solutions "with" or "without" the subject modified by the expressions can both be selected. If the expression "alternative" appears multiple times in a technical solution, unless otherwise specified and without any contradictions or mutually restrictive relationships, the expression "alternative" is independent in each occurrence.

The present invention relates to a preparation method for a magnetic bead, comprising:
preparing a magnetic core by means of a water-soluble salt of Fe²⁺ and Fe³⁺; and
subjecting the magnetic core and a silanide to a modification reaction to obtain the magnetic bead,
wherein the molar ratio of the addition amount of the used silanide to the total amount of Fe²⁺ and Fe³⁺ is greater than or equal to 0.5 : 1. In some embodiments, the molar ratio of the addition amount of the silanide to the total amount of Fe²⁺ and Fe³⁺ is (0.5 : 1)-(4.0 : 1). In other embodiments, the molar ratio of the addition amount of the silanide to the total amount of Fe²⁺ and Fe³⁺ is not less than 0.7 : 1. In some embodiments, the molar ratio of the addition amount of the silanide to the total amount of Fe²⁺ and Fe³⁺ is (0.7 : 1)-(4.0 : 1). In some particular embodiments, the molar ratio of the addition amount of the silanide to the total amount of Fe²⁺ and Fe³⁺ is 0.69 : 1, 0.92 : 1, 1.84 : 1, 2.3 : 1, or 3.70 : 1, or even 5 : 1. In a preferable embodiment, the molar ratio of the addition amount of the silanide to the total amount of Fe²⁺ and Fe³⁺ is 1.84 : 1.

The inventors of the present invention have unexpectedly found that in the preparation of magnetic beads, by increasing the addition amount of a silanide to an excessive amount relative to iron element, the magnetic beads can not only achieve a higher nucleic acid extraction yield, but also effectively increase purified nucleic acids in the supercoiled form.

After a silanide is added to a ferroferric oxide magnetic core formed by Fe²⁺ and Fe³⁺, a silanide-based layer is formed on the surface of the magnetic core, wherein the addition amount may affect the density of silicon hydroxyl. Normally, when the molar ratio of the addition amount of a silanide to the total amount of Fe²⁺ and Fe³⁺ is 0.46, the magnetic core can already be wrapped with a tight silicon hydroxyl layer, by which time the overall loading capacity of the magnetic bead is superior. The inventors speculate that excessive addition may result in excessive silicon hydroxyl enrichment on the surface of this magnetic core. In this way, the overall particle size of the magnetic bead becomes larger, and the surface becomes smoother, with reduced voids, resulting in a change in the nucleic acid extraction performance.

In the present invention, the "impurity-removing magnetic bead" and the "binding magnetic bead" mean that the two are magnetic beads that have different functions in different steps, but the structures and compositions of the two are not limited. Their structures and compositions may be the same or different.

In some embodiments, the silanide comprises at least one of methyl orthosilicate, ethyl orthosilicate, propyl orthosilicate, butyl orthosilicate, alkyltrimethoxysilane, alkyltriethoxysilane, alkyltriisopropylsilane, dialkyldimethoxysilane, dialkyldiethoxysilane, phenyltrimethoxysilane, phenyltriethoxysilane, aminopropyltrimethoxysilane and epoxypropyltrimethoxysilane; and preferably, the silanide comprises at least one of methyl orthosilicate, ethyl orthosilicate, propyl orthosilicate and butyl orthosilicate. In one embodiment, the silanide is ethyl orthosilicate.

In some embodiments, the surface of the magnetic core is treated with a hydrophilic substance prior to the silanide-based modification, wherein the hydrophilic substance is any one or more of citric acid-based, acetate-based, polyethylene glycol-based and polyvinylpyrrolidone-based hydrophilic substances. The treatment with a hydrophilic substance is intended to improve the dispersibility of particles.

Preferably, the citric acid-based hydrophilic substance is a citrate, for example trisodium citrate hydrate or trisodium citrate dihydrate. Preferably, the acetate-based hydrophilic substance is potassium acetate or sodium acetate. In a particular embodiment, the hydrophilic substance is trisodium citrate dihydrate. In another embodiment, the hydrophilic substance is potassium acetate.

In some embodiments, the magnetic core is prepared in the presence of an alkaline pH regulator, and the silanide modification reaction is performed in the presence of an alkaline pH regulator, wherein the pH regulator can be selected from ammonia water, sodium hydroxide, etc.

The method for preparing the magnetic bead may be any method known in the art, such as a chemical coprecipitation method, a solvothermal method, a microemulsion method, a direct-current arc plasma method or a pyrolysis method. The preferred method is a hydrothermal method.

The magnetic core is prepared using a solution of an iron ion salt; the iron ion salt is at least one of a divalent water-soluble salt of iron and a trivalent water-soluble salt of iron; and the solution of the iron ion salt may further contain a salt of any one or more of metal ions of zinc, manganese, titanium, nickel, cobalt, zirconium and chromium.

In some embodiments, the divalent water-soluble salt of iron is one of or a mixture of more of ferrous chloride, ferrous sulphate, ferrous nitrate and hydrates thereof. In some embodiments, the trivalent water-soluble salt of iron is one of or a mixture of more of ferric chloride, ferric sulphate, ferric nitrate and hydrates thereof.

The number of crystal water in the hydrate is not particularly limited. In some more particular embodiments, the water-soluble salt is a mixture of FeCl₃•6H₂O and FeSO₄•7H₂O.

In some embodiments, the molar ratio of Fe²⁺ to Fe³⁺ is (1 : 0.8)-(1 : 3.0), for example, (1 : 1.2)-(1 : 2.2). In a particular embodiment, the molar ratio of Fe²⁺ to Fe³⁺ in the water-soluble salts is 1 : 1.7. In a particular embodiment, Fe²⁺, for example, FeSO₄•7H₂O (0.18 mol) and Fe³⁺, for example, FeCl₃•6H₂O (0.3 mol) are used.

The particle size range of the magnetic bead is preferably 0.1 µm-1mm, e.g., 0.1 µm, 0.5 µm, 1 µm, 2 µm, 5 µm, 10 µm, 20 µm, 50 µm, 100 µm, and 500 µm. In some embodiments, the particle size range of the magnetic bead is 0.1 µm-50 µm, preferably 1 µm-20 µm, and more preferably, the particle size range of the magnetic bead is 1 µm-10 µm.

In some embodiments, a magnetic core is prepared by means of a water-soluble salt of Fe²⁺ and Fe³⁺ in the presence of ammonia water and then subjected to hydrophilic surface modification to obtain a hydrophilic magnetic particle, and a core-shell magnetic bead is synthesized by means of the silanide in the presence of ammonia water.

The water-soluble salt of every about 50 g of Fe²⁺ (for example, FeSO₄•7H₂O, 0.18 mol) or about 82 g of Fe³⁺ (for example, FeCl₃•6HzO, 0.3 mol) corresponds to greater than 50 mL (0.22 mol), greater than or equal to 75 mL (0.34 mol), or greater than or equal to 100 mL (0.44 mol), or greater than or equal to 125 mL (0.56 mol), or 100 mL-400 mL (0.44 mol-1.76 mol), or 125 mL-250 mL (0.56 mol-1.12 mol) of a silanide (especially ethyl orthosilicate). The molar ratio of the addition amount of the silanide to the total amount of Fe²⁺ and Fe³⁺ is respectively 0.69 : 1, 0.92 : 1, 1.84 : 1, 2.3 : 1, or 3.70 : 1.

A still further aspect of the present invention further relates to a magnetic bead prepared by the preparation method for a magnetic bead as described above.

A yet another aspect of the present invention relates to the use of the magnetic bead as described above in nucleic acid extraction.

In some embodiments, the use is achieved through the following steps: adsorbing a nucleic acid to the magnetic bead in a solution system that facilitates the adsorption of the nucleic acid to the modification group of the magnetic bead, and eluting and harvesting the nucleic acid from the magnetic bead.

A still further aspect of the present invention further relates to a method for extracting a nucleic acid, comprising:
providing a biological sample containing a free nucleic acid and non-nucleic acid impurities, an impurity-removing magnetic bead and a binding magnetic bead, wherein the binding magnetic bead is the magnetic bead as described above, and the impurity-removing magnetic bead and the binding magnetic bead are the same or different magnetic bead(s) except that the modification groups on the surface of the two magnetic beads and/or the modification amount of the modification group on the surface of the two magnetic beads are/is different;
adsorbing the non-nucleic acid impurities to the impurity-removing magnetic bead in a solution system that does not facilitate the adsorption of the nucleic acid to the modification group, and removing the impurity-removing magnetic bead; and
adsorbing the nucleic acid to the binding magnetic bead in a solution system that facilitates the adsorption of the nucleic acid to the modification group, and eluting and harvesting the nucleic acid from the binding magnetic bead.

In some embodiments, the modification group of the impurity-removing magnetic bead is selected from silicon hydroxyl, carboxyl or a substance having ion exchange properties. The "substance having ion exchange properties" means that reversible ion exchange can happen between same and the ions in a mobile phase, thereby isolating ionic compounds. Nucleic acids are highly negatively charged linear polyanions, and therefore the substances having ion exchange properties are mostly anion exchange functional groups.

In some embodiments, the substance having ion exchange properties is selected from at least one of amino and imidazolyl.

In some embodiments, the silicon hydroxyl is obtained by silanide modification.

In some embodiments, the silanide comprises at least one of methyl orthosilicate, ethyl orthosilicate, propyl orthosilicate, butyl orthosilicate, alkyltrimethoxysilane, alkyltriethoxysilane, alkyltriisopropylsilane, dialkyldimethoxysilane, dialkyldiethoxysilane, phenyltrimethoxysilane, phenyltriethoxysilane, aminopropyltrimethoxysilane and epoxypropyltrimethoxysilane; and preferably, the silanide comprises at least one of methyl orthosilicate, ethyl orthosilicate, propyl orthosilicate and butyl orthosilicate.

The silanide-modified impurity-removing magnetic bead is low in preparation cost, has both good impurity-removing effects and high impurity-loading capacity, and can remove impurities such as cell debris and tissue fragments with high efficiency, no longer subject to the limitations of centrifugation and filtration, and showing obvious advantages in nucleic acid purification.

In some embodiments, the molar ratio of the silanide to the total amount of Fe²⁺ and Fe³⁺ is less than or equal to 0.3, e.g., 0.25, 0.2, 0.15, or 0.1.

In some embodiments, the preparation method for the impurity-removing magnetic bead comprises:
preparing a magnetic core by means of a water-soluble salt of Fe²⁺ and Fe³⁺, subjecting the magnetic core to hydrophilic surface modification to obtain a hydrophilic magnetic particle, and synthesizing a core-shell magnetic bead by means of the silanide in the presence of ammonia water,
wherein the water-soluble salt of every about 100 g of Fe²⁺ (for example, FeSO_{4*}7H₂O) or 164 g of Fe³⁺ (for example, FeCl₃•6H₂O) corresponds to 25 mL-50 mL (e.g., 30 mL, 40 mL, 45 mL), or 30 mL-70 mL, or 40 mL-60 mL of a silanide (especially ethyl orthosilicate).

The particle size range of the impurity-removing magnetic bead is preferably 0.1 µm-1 mm, e.g., 0.1 µm, 0.5 µm, 1 µm, 2 µm, 5 µm, 10 µm, 20 µm, 50 µm, 100 µm, and 500 µm. In some embodiments, the particle size range of the impurity-removing magnetic bead is 0.1 µm-50 µm, preferably 1 µm-20 µm, and more preferably, the particle size range of the magnetic bead is 1 µm-10 µm.

In some embodiments, a sufficient amount of an alcohol and/or a chaotropic salt is not present in the solution system that does not facilitate the adsorption of the nucleic acid to the modification group; and
a sufficient amount of an alcohol and/or a chaotropic salt is present in the solution system that facilitates the adsorption of the nucleic acid to the modification group.

In some embodiments, the alcohol comprises one or more of isopropyl alcohol, propanol, butanol, methanol and absolute ethanol.

In some embodiments, the chaotropic salt comprises one or more of guanidine isothiocyanate, guanidine isocyanate, guanidine thiocyanate, guanidine hydrochloride, sodium iodide, lithium acetate, lithium chloride, lithium perchlorate and sodium perchlorate.

In the present invention, there is no intended difference in length between the terms "nucleic acid" and "oligonucleotide", both of which are N-glycosides of purine or pyrimidine bases, or modified purine or pyrimidine bases. These terms refer only to the primary structure of molecules. Thus, these terms include double-stranded and single-stranded DNA, as well as double-stranded and single-stranded RNA, and double-stranded RNA-DNA hybrids. In some embodiments, the nucleic acid comprises at least one of plasmid DNA, genomic DNA, mitochondrial DNA, chloroplast DNA, a DNA fragment, total RNA, pre-mRNA, mRNA, rRNA, tRNA, ncRNA, snRNA, 1ncRNA and miRNA. In one embodiment, the nucleic acid is plasmid DNA.

In some embodiments, the biological sample is from an animal, a plant or a microorganism.

The animal may be a mammal, preferably a primate, and more preferably a rat, a rabbit, a cow, a horse, a sheep, a dog, and a human; and may also be nematodes. The animal may also be an insect, for example, a fruit fly. The plant is for example, *Arabidopsis thaliana,* rice, wheat, corn, soybean, and cotton. The microorganism can be a virus, a bacterium, or a fungus (e.g., yeast).

Any biological sample containing a free nucleic acid can be used in the method provided by the present disclosure. Free nucleic acid means that the nucleic acid can directly come in contact with a solid phase (for example, a magnetic bead) without the need for additional processes such as lysis or release. It may be a solution containing one or more molecules (for example, polypeptides or nucleic acids) derived from cells, cellular materials or viral materials; or a solution containing naturally occurring or non-naturally occurring nucleic acids. In some embodiments, the biological sample can be treated by a chemical method, a physical method, or an enzymatic method, etc. to release nucleic acids. In some embodiments, the non-nucleic acid impurities include one or more of cell debris, tissue fragments, blood clots, proteins, lipids, polysaccharides and salt ions, and may be insoluble and/or soluble impurities. In some embodiments, the sample may also be from a body fluid, including liquid, semi-solid, aerated liquid, liquid-gas mixture, etc., from an animal. Such body fluids may include, but are not limited to, saliva, sputum, serum, plasma, blood, urine, mucus, sweat, tears or other ocular fluids, otic fluids, facial fluid (such as fluid from blisters or sores), gastric fluids or gastric juices, faecal fluids, pancreatic fluids or juices, semen, products of lactation or mensuration, spinal fluids, liquid bone marrow or lymph fluid. In some embodiments, the biological sample is a tissue lysate or a cell lysate.

The present invention further relates to a nucleic acid extraction kit comprising the magnetic bead as described above.

In some embodiments, the kit further comprises an impurity-removing magnetic bead, wherein the modification group of the impurity-removing magnetic bead is silicon hydroxyl, carboxyl or a substance having ion exchange properties.

The modification group of the impurity-removing magnetic bead is selected from at least one of silicon hydroxyl, carboxyl and amino; and the silicon hydroxyl is obtained by silanide modification; preferably, the silanide comprises at least one of methyl orthosilicate, ethyl orthosilicate, propyl orthosilicate, butyl orthosilicate, alkyltrimethoxysilane, alkyltriethoxysilane, alkyltriisopropylsilane, dialkyldimethoxysilane, dialkyldiethoxysilane, phenyltrimethoxysilane, phenyltriethoxysilane, aminopropyltrimethoxysilane and epoxypropyltrimethoxysilane, and more preferably, the silanide comprises at least one of methyl orthosilicate, ethyl orthosilicate, propyl orthosilicate and butyl orthosilicate.

In some embodiments, the kit further comprises one or more of the following ingredients:
lysis buffer: for lysing a biological sample and releasing a nucleic acid contained therein;
neutralizing solution: for terminating the lysis reaction of the lysis buffer;
binding buffer: comprising an alcohol and/or a chaotropic salt that facilitates the adsorption of the nucleic acid to the binding magnetic bead;
wash buffer: for washing away impurities other than the nucleic acid on the binding magnetic bead;
elution buffer: for eluting the nucleic acid from the binding magnetic bead.

In some embodiments, the kit further comprises one or more of the following ingredients:
solution A: containing 10 mM-50 mM EDTA, Tris-HCl and 50 µg/mL-100 µg/mL RNaseA, pH = 7.5-8.5;
solution B: containing 0.5 M-1 M NaOH and 1 w/v%-10 w/v% SDS;
solution C: containing 3.0 M-4.0 M potassium acetate, pH = 5.2-5.5;
solution D: containing 2 M-3 M guanidine hydrochloride;
solution E: an aqueous solution containing 70 v/v%-80 v/v% ethanol;
solution F: containing 5 nM-15 mM Tris-HCl and 0.5 mM-1.5 mM EDTA, pH = 7.5-8.5.

A still further aspect of the present invention relates to a method for extracting a nucleic acid, comprising: providing a biological sample containing a free nucleic acid and non-nucleic acid impurities; adsorbing the non-nucleic acid impurities to an impurity-removing magnetic bead; and removing the impurity-removing magnetic bead by adding a magnetic field to obtain a solution comprising the nucleic acid, wherein the impurity-removing magnetic bead comprises silicon hydroxyl, carboxyl or a modification group having ion exchange properties.

In some embodiments, the modification group of the impurity-removing magnetic bead is selected from at least one of silicon hydroxyl, carboxyl and amino; and preferably, the modification group is silicon hydroxyl. In one embodiment, the modification group of the impurity-removing magnetic bead is silicon hydroxyl.

In some embodiments, the silicon hydroxyl is obtained by silanide modification. Preferably, the silanide comprises at least one of methyl orthosilicate, ethyl orthosilicate, propyl orthosilicate, butyl orthosilicate, alkyltrimethoxysilane, alkyltriethoxysilane, alkyltriisopropylsilane, dialkyldimethoxysilane, dialkyldiethoxysilane, phenyltrimethoxysilane, phenyltriethoxysilane, aminopropyltrimethoxysilane and epoxypropyltrimethoxysilane. In some preferred embodiments, the silanide comprises at least one of methyl orthosilicate, ethyl orthosilicate, propyl orthosilicate and butyl orthosilicate. In one embodiment, the silanide is ethyl orthosilicate.

The above-mentioned definition of the impurity-removing magnetic bead in the present invention is applicable to the impurity-removing magnetic bead in the method.

In some embodiments, the method further comprises: adding a binding magnetic bead to the solution comprising the nucleic acid, and adsorbing the nucleic acid in the solution system that facilitates the adsorption of the nucleic acid to the binding magnetic bead; and eluting and harvesting the nucleic acid from the binding magnetic bead, wherein the binding magnetic bead is selected from a magnetic bead comprising at least one of a silicon hydroxyl modification group, a carboxyl modification group and an amino modification group. Preferably, the binding magnetic bead is a magnetic bead comprising a silicon hydroxyl modification group.

In some embodiments, the binding magnetic bead is obtained by performing silanide modification on the surface of the magnetic core prepared by means of iron element, wherein the molar ratio of the silanide to the total amount of Fe²⁺ and Fe³⁺ is (0.5 : 1)-(4.0 : 1). In a preferred embodiment, the molar ratio of the silanide to the total amount of Fe²⁺ and Fe³⁺ is (0.7 : 1)-(4.0 : 1), preferably (0.92 : 1)-(2.3 : 1).

In some embodiments, the molar ratio of Fe²⁺ to Fe³⁺ is (1 : 0.8)-(1 : 3.0), preferably 1 : 1.7.

A sufficient amount of an alcohol and/or a chaotropic salt is present in the solution system that facilitates the adsorption of the nucleic acid to the binding magnetic bead.

In some embodiments, the alcohol comprises one or more of isopropyl alcohol, propanol, butanol, methanol and absolute ethanol.

In some embodiments, the chaotropic salt comprises one or more of guanidine isothiocyanate, guanidine isocyanate, guanidine thiocyanate, guanidine hydrochloride, sodium iodide, lithium acetate, lithium chloride, lithium perchlorate and sodium perchlorate.

Other definitions and/or preparation methods, etc. involving the biological sample, nucleic acid and magnetic core in the present invention are all applicable to the present method, and will not be repeated here.

The present invention relates to a method for extracting and purifying a high-purity target nucleic acid on the basis of an impurity-removing magnetic bead and a binding magnetic bead, wherein the method may comprise the following steps:
(1) cell lysis: disrupting cells or the cell structure of biological tissues by a chemical method, a physical method, or an enzymatic method, etc. to obtain a solution containing all the biological materials;
(2) impurity adsorption: mixing the solution with the impurity-removing magnetic bead, and selectively adsorbing the biological materials except the target nucleic acid in the solution to the magnetic microspheres to form complex I;
(3) impurity removal: isolating complex I from the solution by applying a magnetic field so as to obtain a supernatant containing the target nucleic acid;
(4) nucleic acid adsorption: mixing the above-mentioned supernatant containing the target nucleic acid with magnetic microspheres in the presence of an alcohol or a chaotropic salt, by which time the target nucleic acid in the solution and the magnetic microspheres form complex II;
(5) washing: washing the nucleic acid/magnetic bead complex II with a washing buffer under the action of an external magnetic field so as to remove the impurities such as residual salt ions in the solution;
(6) nucleic acid recovery: desorbing the target nucleic acid on the complex using an elution buffer, and removing the binding magnetic bead by applying an external magnetic field to obtain a solution containing the target nucleic acid.

The present invention has the following beneficial effects:
(1) The impurity-removing magnetic bead used in the present invention can be used to quickly and effectively remove the biological materials (such as cell debris, tissue fragments and abundant proteins and lipids contained therein) except the target nucleic acid from various samples such as cells, tissues and blood, thereby facilitating the subsequent nucleic acid purification process.
(2) The method for extracting a nucleic acid provided by the present invention can be used to quickly purify the nucleic acids and achieve high nucleic acid purification yield, good purity and integrity. Moreover, by means of the method, impurity removal is no longer subject to the limitations of centrifugation or suction filtration, and its effects in plasmid purification is particularly significant, effectively enabling the fully automated nucleic acid purification of various samples.

The embodiments of the present invention will be described in detail below with reference to the examples. It should be understood that these examples are merely intended to illustrate the present invention but not to limit the scope of the present invention. For the experimental methods in the following examples, if no specific conditions are specified, priority is given to the guidance provided in this invention, and they can also be performed according to the experimental manual or conventional conditions in the art, other experimental methods known in the art, or the conditions suggested by the manufacturer.

In the following specific examples, the measurement parameters related to the components of the raw materials may have slight deviations within the weighing accuracy, unless otherwise specified. When temperature and time parameters are involved, acceptable deviations due to instrument testing accuracy or operating accuracy are allowed.

### Example 1 Preparation of impurity-removing magnetic bead and related tests

This example is intended to illustrate the preparation process of the impurity-removing magnetic bead of the present invention while providing data related to the magnetic bead.

### 1. Preparation of impurity-removing magnetic bead

(1) 164 g of FeCl₃•6H₂O and 100 g of FeSO₄•7H₂O were weighed and dissolved in an appropriate amount of deoxygenated water respectively; after dissolution, the resulting solutions were added to a three-neck flask, and stirred and uniformly mixed; and 100 mL of 200 g/L KOAc catalyst was added into the flask, and the mixture was reacted for 30-60 min in a water bath at 60°C-90°C continuously.
(2) 50 mL of ammonia water was added, and the resulting mixture was continuously stirred for 10-20 min in the above-mentioned water bath at 60°C-90°C to harvest a magnetic core.
(3) The above-mentioned magnetic core was washed with deionized water, and then subjected to ultrasonic treatment by adding 2 L of absolute ethanol.
(4) At room temperature, 1 L of 1% sodium citrate dihydrate was added to the above solution, and the resulting mixture was continuously and uniformly mixed for 10-30 min.
(5) After the mixing, 100 mL of ethyl orthosilicate (Sinopharm, Cat. No. 78-10-4) was slowly added, and the mixture was continuously stirred for 6 h.
(6) The magnetic microspheres were harvested, washed multiple times with deionized water, and stored at room temperature.

### 2. Physicochemical property test of impurity-removing magnetic bead

The particle size and degree of dispersion of the magnetic bead were determined using a Malvern particle size analyser (GR13010266, Malvern). The specific steps are as follows: the magnetic bead to be tested was uniformly mixed by shaking; then 900 mL of deionized water was added into a measuring beaker; "start" was clicked to perform background measurement; after the background measurement was completed, 300 uL of the magnetic bead sample was added into the beaker; and "start" was clicked to complete the measurement of the particle size and degree of dispersion of the sample.

As shown in FIG. 1 and Table 1, the impurity-removing magnetic bead provided by the present invention had an average particle size of 5.496 µm, a particle size range of 2-20 µm, and a degree of dispersion of 0.351, indicating that the magnetic bead had a good particle size and homogeneity.

**Table 1 Particle size and degree of dispersion determination results of impurity-removing magnetic bead**

| Type of magnetic bead | Particle size (µm) | Degree of dispersion |
|---|---|---|
| Impurity-removing magnetic bead | 5.496 | 0.351 |

### 3. Impurity removal property test of impurity-removing magnetic bead

### Formulation of purification reagent

Resuspension solution S1: a mixed solution consisting of 10-50 mM EDTA, Tris-HCl and 50-100 µg/mL RNaseA, pH 8.0
Lysis buffer S2: a mixed solution consisting of 0.5-1 M NaOH and 1%-10% SDS
Neutralizing solution S3: a solution consisting of 3.0-4.0 M KOAc, pH 5.2-5.5

(1) 0.6 g, 0.8 g, 1.0 g, 1.2 g and 1.5 g of bacterial sludge were respectively weighed and added into five 50 mL centrifuge tubes, and 12 mL of solution S1 was added into each centrifuge tube to fully resuspend the cells.
(2) 12 mL of solution S2 was added to the above samples, respectively; the resulting mixtures were each uniformly mixed by gently turning the tubes upside down 6 times and allowed to stand for 4 min; and 12 mL of solution S3 was added to each sample, and the resulting mixtures were each uniformly mixed by quickly and gently turning the tubes upside down 20 times.
(3) 125 mg of the impurity-removing magnetic bead was added into each tube, and the resulting mixtures were each uniformly mixed by repeatedly turning the tubes upside down for 1 min.
(4) The above-mentioned five 50 mL centrifuge tubes were placed on a magnetic stand and allowed to stand until the solutions were clear; the supernatants were removed; and the impurity/impurity-removing magnetic bead complex was dried in an oven at 37°C and then weighed, and the weight of the impurities was calculated.

As shown in Table 2, the studies showed that 125 mg of the impurity-removing magnetic bead can be used to remove at most about 1.3 g of cellular impurities, and could meet the test requirement of 1.5 g of bacterial sludge sample, indicating that the impurity-removing magnetic bead had high removal efficiency and was no longer subject to the limitations of centrifugation and suction filtration.

**Table 2 Impurity removal capability test of impurity-removing magnetic bead**

| Cell amount (g) | Addition amount of magnetic bead (mg) | Amount of removed impurity (g) |
|---|---|---|
| 0.6 | 125 | 0.53 |
| 0.8 | 125 | 0.71 |
| 1.0 | 125 | 0.88 |
| 1.2 | 125 | 1.01 |
| 1.5 | 125 | 1.31 |

When the cell amount is greater than 1.5 g, the complex formed by the impurities/magnetic bead cannot be completely isolated under the action of a magnetic field due to excessive impurities, resulting in that the supernatant containing the target nucleic acid cannot be effectively isolated, unless the amount of impurities contained in the cells is less than 1.3 g (the content of the impurities was directly related to the species, culture method, etc. of the bacterium). At this time, it is necessary to continue to add a certain proportion of the impurity-removing magnetic beads based on the impurity removal efficiency of the impurity-removing magnetic bead.

### Example 2 Preparation of binding magnetic bead and related test

This example is intended to illustrate the preparation process of the binding magnetic bead of the present invention while providing data related to the physicochemical properties of the magnetic bead.

### 1. Preparation of binding magnetic bead

(1) 82 g of FeCl₃•6H₂O and 50 g of FeSO₄•7H₂O were taken and dissolved in 500 mL of deoxygenated water respectively; the two solutions were then sequentially added into a 2 L three-neck flask, and stirred and uniformly mixed; and then 50 mL of 200 g/L KOAc catalyst was added, and the mixture was continuously reacted for 30 min in a water bath at 60°C-90°C.
(2) 10 mL of ammonia water was added, and the resulting mixture was continuously stirred for 10-20 min in a water bath at 60°C to harvest a magnetic core.
(3) The above solution was washed 3 times with deionized water, and then subjected to ultrasonic treatment for 20 min by adding 800 mL of absolute ethanol.
(4) At room temperature, 500 mL of 1% sodium citrate dihydrate was added to the above solution, and the resulting mixture was continuously and uniformly mixed for 10 min.
(5) After 10 mL of ammonia water was added and the resulting mixture was uniformly mixed, 125 mL of ethyl orthosilicate (Sinopharm, Cat. No. 78-10-4) was slowly added, and the mixture was continuously stirred overnight.
(6) The magnetic microspheres were harvested, washed multiple times with deionized water, and stored at room temperature.

### 2. Physicochemical property test of binding magnetic bead

The prepared binding magnetic beads were tested for related properties, such as particle size and degree of dispersion, and the method for testing the particle size and degree of dispersion of the binding magnetic beads was the same as step 2 of Example 1.

As shown in FIG. 1 and Table 3, the binding magnetic bead provided by the present invention had an average particle size of 3.099 µm, a particle size range of 1-10 µm, and a degree of dispersion of 0.358, indicating good uniformity, and the binding magnetic bead had a smaller particle size compared with the impurity-removing magnetic bead.

**Table 3 Particle size and degree of dispersion determination results of binding magnetic bead**

| Type of magnetic bead | Particle size (µm) | Degree of dispersion |
|---|---|---|
| Binding magnetic bead | 3.099 | 0.358 |

### Example 3 Effect of addition amount of ethyl orthosilicate in binding magnetic bead on supercoiled conformation of plasmids

This example is intended to demonstrate the effect of the addition amount of ethyl orthosilicate in the preparation of the binding magnetic bead on the supercoiled conformation of plasmids.

### 1. Formulation of purification reagent

Resuspension solution S1: a mixed solution consisting of 10-50 mM EDTA, Tris-HCl and 50-100 µg/mL RNaseA, pH 8.0
Lysis buffer S2: a mixed solution consisting of 0.5-1 M NaOH and 1%-10% SDS
Neutralizing solution S3: a solution consisting of 3.0-4.0 M KOAc, pH 5.2-5.5
Binding buffer: 2-3 M guanidine hydrochloride solution
Wash buffer: 75% ethanol solution
Elution buffer: a mixed solution consisting of 10 mM Tris-HCl and 1 mM EDTA, pH 8.0

### 2. Preparation of four binding magnetic beads

The four binding magnetic beads were prepared according to the method for preparing the binding magnetic bead in Example 2, wherein the addition amounts of ethyl orthosilicate (Sinopharm, Cat. No. 78-10-4) in step 1.5 were 50 mL, 75 mL, 100 mL and 125 mL, respectively.

### 3. Plasmid extraction

Plasmid extraction was completed with the above four binding magnetic beads according to the following steps:
(1) 1.2 g of bacterial sludge was respectively weighed and added into four 50 mL centrifuge tubes, and 12 mL of solution S1 was added into each centrifuge tube to fully resuspend the cells.
(2) 12 mL of solution S2 was added to the above samples, respectively; the resulting mixtures were each uniformly mixed by gently turning the tubes upside down 6 times and allowed to stand for 4 min; and 12 mL of solution S3 was added to each sample, and the resulting mixtures were each uniformly mixed by quickly and gently turning the tubes upside down 20 times.
(3) 2.5 mL of the impurity-removing magnetic bead (solid content: 50 mg/mL) was added into each tube, and the resulting mixtures were each uniformly mixed by repeatedly turning the tubes upside down for 1 min.
(4) The above four 50 mL centrifuge tubes were placed on a magnetic stand and allowed to stand until the solutions were clear, and the supernatants in the centrifuge tubes were carefully transferred to four new 50 mL centrifuge tubes, respectively. 5 mL of binding buffer was added to each tube, 2.5 mL (solid content: 50 mg/mL) of the above-mentioned four different binding magnetic beads were added, respectively, and the tubes were repeatedly turned upside down for 1 min.
(5) All the above-mentioned 50 mL centrifuge tubes were placed on the magnetic stand to adsorb the magnetic beads, the liquids were poured off, 10 mL of wash buffer was added to each centrifuge tube, and the resulting mixtures were each uniformly mixed by manually and repeatedly inverting the tubes for 1 min.
(6) All the above-mentioned 50 mL centrifuge tubes were placed on the magnetic stand to adsorb the magnetic beads, the liquids were poured off, the liquids at the bottoms of the tubes were aspirated and discarded, and the tubes were allowed to stand for about 5-10 min so as to remove the residual ethanol.
(7) The above samples were taken from the magnetic stand, 1.5 mL of elution buffer was added into each tube, the resulting mixtures were each uniformly mixed by vortexing, the tubes were then allowed to stand on the magnetic stand to adsorb the magnetic beads for 1 min, and the liquids were transferred to four new 1.5 mL centrifuge tubes respectively.

In the experiment, a commercial plasmid extraction kit (Qiagen, Cat. No. 12262) was used as a positive control, and agarose gel electrophoresis was used to detect the proportion of supercoiled plasmids, wherein lanes 1-4 represented the plasmid samples purified with the four binding magnetic beads prepared with 50 mL, 75 mL, 100 mL and 125 mL of ethyl orthosilicate additions, respectively, and lane 5 represented the plasmid sample obtained with the commercial plasmid extraction kit. The addition amounts of the plasmid samples were all 50 ng, 10 µL, and Marker was purchased from New England Biolabs (Cat. No. N3232L).

As shown in FIG. 2 and Table 4, with the increase in the addition amount of ethyl orthosilicate in the magnetic bead preparation process, the proportion of the supercoiled plasmids in the purified plasmids increased significantly. When the addition amount of ethyl orthosilicate was 125 mL, the proportion of the supercoiled plasmids reached 95% or more, which was consistent with that of the commercial plasmid extraction kit. The result indicated that the binding magnetic bead provided by the present invention could effectively increase purified nucleic acids in the supercoiled form, where the key control point was in the addition level of ethyl orthosilicate in the binding magnetic bead preparation process.

**Table 4 Determination of proportion of supercoiled plasmids**

| Group | Proportion of supercoiled plasmids (%) |
|---|---|
| Ethyl orthosilicate-50 mL | 63.563 |
| Ethyl orthosilicate-75 mL | 86.967 |
| Ethyl orthosilicate-100 mL | 93.473 |
| Ethyl orthosilicate-125 mL | 97.122 |
| Commercial plasmid extraction kit | 96.231 |

### Example 4 Effect of excessive ethyl orthosilicate added in binding magnetic bead on supercoiled conformation of plasmids

This example is intended to demonstrate the effect of excessive ethyl orthosilicate added in the preparation of the binding magnetic bead on the supercoiled conformation of plasmids.

In the example, the amount of ethyl orthosilicate was increased to 150 mL, 200 mL, and 250 mL, the specific steps for preparing the binding magnetic bead were the same as those in Example 2, and the steps for isolating and purifying the plasmids were the same as those in Example 3. In the experiment, a commercial plasmid extraction kit (Qiagen, Cat. No. 12262) was used as a positive control, and agarose gel electrophoresis was used to detect the proportion of supercoiled plasmids, wherein the addition amounts of plasmid samples in lanes 1-4 were all 50 ng, 10 µL, and Marker was purchased from New England Biolabs (Cat. No. N3232L).

As shown in FIG. 3 and Table 5, when the addition amount of ethyl orthosilicate in the preparation of the binding magnetic bead reached 150 mL or more, the proportion of the supercoiled plasmids remained high and was substantially consistent with that of the commercial plasmid extraction kit. This result indicated that in the step for preparing the binding magnetic bead, with the excessive addition of ethyl orthosilicate, the binding magnetic bead still had good plasmid purification properties, and the proportion of the supercoiled plasmids in the purified plasmids could well satisfy downstream study.

**Table 5 Determination of proportion of supercoiled plasmids**

| Group | Proportion of supercoiled plasmids (%) |
|---|---|
| Ethyl orthosilicate-150 mL | 94.692 |
| Ethyl orthosilicate-200 mL | 94.836 |
| Ethyl orthosilicate-250 mL | 94.631 |
| Commercial plasmid extraction kit | 93.807 |

### Example 5 Test of adsorption capacity of binding magnetic bead to plasmid DNA

This example is intended to demonstrate the high adsorption capacity of the binding magnetic bead provided by the present invention to the target nucleic acid.

The specific steps of the Example are as follows:
(1) 1.2 g of bacterial sludge was weighed and added into a 50 mL centrifuge tube, and 12 mL of solution S1 was added into the centrifuge tube to fully resuspend the cells.
(2) 12 mL of solution S2 was added to the sample; the resulting mixture was uniformly mixed by gently turning the tube upside down 6 times and allowed to stand for 4 min; and 12 mL of solution S3 was added to the sample, and the resulting mixture was uniformly mixed by quickly and gently turning the tube upside down 20 times.
(3) 2.5 mL of the impurity-removing magnetic bead (solid content: 50 mg/mL) was added, and the resulting mixture was uniformly mixed by repeatedly turning the tube upside down for 1 min.
(4) The above 50 mL centrifuge tube was placed on a magnetic stand and allowed to stand until the solution was clear, and the supernatant in the centrifuge tube was carefully transferred to nine new 15 mL centrifuge tubes (3.5 mL each).
(5) 0.66 mL of binding buffer was added to each of the above-mentioned 15 mL centrifuge tubes. After the resulting mixtures were uniformly mixed, 125 uL, 250 uL and 500 uL of the binding magnetic beads (2 different batches, solid content: 50 mg/mL) prepared in Example 2 and a commercial magnetic bead (solid content: 50 mg/mL, BioSune Biotechnology (Shanghai) Co., Ltd., BS010100) were added respectively, and the resulting mixtures were uniformly mixed by repeatedly turning the tubes upside down for 1 min.
(7) All the above-mentioned 15 mL centrifuge tubes were placed on the magnetic stand to adsorb the magnetic beads, the liquids were poured off, 3 mL of wash buffer was added to each centrifuge tube, and the resulting mixtures were each uniformly mixed by manually and repeatedly inverting the tubes for 1 min.
(8) All the above-mentioned 15 mL centrifuge tubes were placed on the magnetic stand to adsorb the magnetic beads, the liquids were poured off, the liquids at the bottoms of the tubes were aspirated and discarded, and the tubes were allowed to stand for about 5-10 min so as to remove the residual ethanol.
(9) The above samples were taken from the magnetic stand, 0.5 mL of elution buffer was added into each tube, the resulting mixtures were each uniformly mixed by vortexing, the tubes were then allowed to stand on the magnetic stand to adsorb the magnetic beads for 1 min, and the liquids were transferred to new 1.5 mL centrifuge tubes.

The results as shown in FIG. 4 indicated that the performance of the magnetic beads of the present invention was relatively stable between different batches. Moreover, with the increase of the amount of the binding magnetic bead, the plasmid-loading capacity of the binding magnetic beads of the present invention was constantly increased. When the addition amount of the binding magnetic bead was 25 mg, the amount of the adsorbed plasmids could reach 500-600 µg, that is, each mg of the binding magnetic bead could adsorb about 20 µg of the plasmid. Additionally, the plasmid-loading capacity of the binding magnetic bead of the present invention was about 1.5-2 times that of the commercial magnetic bead at all the different amounts, indicating that the plasmid adsorption capacity of the binding magnetic bead of the present invention was much greater than that of the commercial magnetic bead. These results demonstrated that the binding magnetic bead of the present invention had a significantly greater nucleic acid adsorption capacity and could well meet the requirements of nucleic acid purification.

### Example 6 Plasmid purification test based on impurity-removing magnetic bead and binding magnetic bead

This example is intended to illustrate the use and performance of the impurity-removing magnetic bead and binding magnetic bead provided by the present invention in methods for purifying plasmids.

The specific steps of the Example are as follows:
(1) 1.2 g of each of four different strains (DH5a, TOP10, BL21, JM109) was weighed and added into four 50 mL centrifuge tubes respectively, and 12 mL of solution S1 was added into each centrifuge tube to fully resuspend the cells.
(2) 12 mL of solution S2 was added to each sample; the resulting mixtures were uniformly mixed by gently turning the tubes upside down 6 times and allowed to stand for 4 min; and 12 mL of solution S3 was added to each sample, and the resulting mixtures were uniformly mixed by quickly and gently turning the tube upside down 20 times.
(3) 2.5 mL of the impurity-removing magnetic bead (solid content: 50 mg/mL) of Example 1 was added into each tube, and the resulting mixtures were each uniformly mixed by inverting the tubes upside down for 1 min.
(4) The above four 50 mL centrifuge tubes were placed on a magnetic stand and allowed to stand until the solutions were clear, and the supernatants in the centrifuge tubes were carefully transferred to four new 50 mL centrifuge tubes, respectively. 5 mL of binding buffer and 2.5 mL of the binding magnetic beads prepared in Example 2 were added, and the tubes were each repeatedly turned upside down for 1 min.
(5) All the above-mentioned 50 mL centrifuge tubes were placed on the magnetic stand to adsorb the magnetic beads, the liquids were poured off, 10 mL of wash buffer was added to each centrifuge tube, and the resulting mixtures were each uniformly mixed by manually and repeatedly inverting the tubes for 1 min.
(6) All the above-mentioned 50 mL centrifuge tubes were placed on the magnetic stand to adsorb the magnetic beads, the liquids were poured off, the liquids at the bottoms of the tubes were aspirated and discarded, and the tubes were allowed to stand for about 5-10 min so as to remove the residual ethanol.
(7) The above samples were taken from the magnetic stand, 1.5 mL of elution buffer was added into each tube, the resulting mixtures were each uniformly mixed by vortexing, the tubes were then allowed to stand on the magnetic stand to adsorb the magnetic beads for 1 min, and the liquids were transferred to four new 1.5 mL centrifuge tubes respectively.

In the experiment, a commercial plasmid extraction kit (Qiagen, Cat. No. 12262) was used as a positive control, and plasmids were extracted from the four strains (strain 1#, strain 2#, strain 3# and strain 4#) according to the method in the instructions.

The concentration, OD_{260/280} ratio and OD_{260/230} ratio of each purified plasmid were determined using Nanodrop, and the proportion of supercoiled plasmids in the purified plasmid samples was detected by agarose gel electrophoresis.

As shown in Table 6, the plasmid yield of the commercial plasmid extraction kit was about 900-1100 µg, and the yield of the plasmid extracted by the method provided by the present invention was about 1300-1600 µg. According to the quality standard of the plasmid, the OD_{260/280} ratio should be between 1.8-2.0, and the OD_{260/230} ratio should be greater than 2.0. The experimental results showed that the purity of both the plasmid DNA purified with the commercial plasmid extraction kit and the plasmid DNA purified by the method provided by the present invention reached the standard.

As shown in FIG. 5 and Table 7, the proportions of supercoiled plasmids in both the plasmid DNA purified with the commercial plasmid extraction kit and the plasmid DNA purified by the method provided by the present invention were substantially consistent, and both reached about 95%. In terms of experimental operation time, it took 2.5 h to complete the purification with the commercial plasmid extraction kit, whereas it took 1.5 h to complete the purification by the magnetic bead-based purification method provided by the present invention.

According to the above-mentioned results, the magnetic bead-based purification method for plasmid DNA provided by the present invention achieved a higher plasmid DNA extraction yield than the commercial plasmid extraction kit, its plasmid DNA purity and supercoiled plasmid proportion were substantially consistent with those of the commercial plasmid extraction kit, and both of them met the quality standards. In addition, the plasmid DNA purification method provided by the present invention had higher efficiency, was no longer subject to the limitations of centrifugation and suction filtration, and was more convenient to operate, thereby achieving automated purification in a better fashion.

**Table 6 Determination of plasmid concentration, OD_{260/280} ratio and OD_{260/230} ratio**

| No. | Sample | Extraction method | Concentration (ng/µL) | Elution volume | OD260/ 280 | OD260/2 30 |
|---|---|---|---|---|---|---|
| 1 | Strain 1# | Commercial plasmid extraction kit | 654 | 1.5 mL | 1.91 | 2.33 |
| 2 | Strain 2# | Commercial plasmid extraction kit | 737.3 | 1.5 mL | 1.92 | 2.34 |
| 3 | Strain 3# | Commercial plasmid extraction kit | 716.8 | 1.5 mL | 1.93 | 2.32 |
| 4 | Strain 4# | Commercial plasmid extraction kit | 626.9 | 1.5 mL | 1.93 | 2.31 |
| 5 | Strain 1# | Isolation and purification method based on impurity-removing magnetic bead | 1156.7 | 1.5 mL | 1.87 | 2.44 |
| 6 | Strain 2# | Isolation and purification method based on impurity-removing magnetic bead | 1053.3 | 1.5 mL | 1.88 | 2.45 |
| 7 | Strain 3# | Isolation and purification method based on impurity-removing magnetic bead | 1084.5 | 1.5 mL | 1.88 | 2.45 |
| 8 | Strain 4# | Isolation and purification method based on impurity-removing magnetic bead | 876.9 | 1.5 mL | 1.87 | 2.44 |

**Table 7 Determination of proportion of supercoiled plasmids**

| No. | Proportion of supercoiled plasmids (%) |
|---|---|
| 1 | 97.067 |
| 2 | 96.583 |
| 3 | 97.268 |
| 4 | 98.177 |
| 5 | 90.887 |
| 6 | 95.455 |
| 7 | 93.031 |
| 8 | 96.236 |

The above examples merely represent several embodiments of the present invention, giving specifics and details thereof, but should not be understood as limiting the scope of the present patent of invention thereby. It should be noted that those of ordinary skill in the art could also make several alterations and improvements without departing from the spirit of the present invention and these would all fall within the scope of protection of the present invention. Therefore, the scope of protection of the present patent of invention shall be in accordance with the appended claims, and the description and accompanying drawings can be used to explain the content of the claims.

## Claims

1. A preparation method for a magnetic bead, **characterised in that** the preparation method for a magnetic bead comprises:
preparing a magnetic core by means of a water-soluble salt of Fe²⁺ and Fe³⁺; and
subjecting the magnetic core and a silanide to a modification reaction to obtain the magnetic bead,
wherein the molar ratio of the addition amount of the used silanide to the total amount of Fe²⁺ and Fe³⁺ is greater than or equal to 0.5 : 1, preferably (0.5 : 1)-(4.0 : 1), and the particle size range of the magnetic bead is 0.1 µm-50 µm.

2. The preparation method for a magnetic bead according to claim 1, **characterised in that** the molar ratio of the addition amount of the silanide to the total amount of Fe²⁺ and Fe³⁺ is (0.7 : 1)-(4.0 : 1), preferably (0.92 : 1)-(2.3 : 1).

3. The preparation method for a magnetic bead according to claim 1 or 2, **characterised in that** the molar ratio of the Fe²⁺ to the Fe³⁺ is (1 : 0.8)-(1 : 3.0), preferably 1 : 1.7.

4. The preparation method for a magnetic bead according to claim 1, **characterised in that** the silanide comprises at least one of methyl orthosilicate, ethyl orthosilicate, propyl orthosilicate, butyl orthosilicate, alkyltrimethoxysilane, alkyltriethoxysilane, alkyltriisopropylsilane, dialkyldimethoxysilane, dialkyldiethoxysilane, phenyltrimethoxysilane, phenyltriethoxysilane, aminopropyltrimethoxysilane and epoxypropyltrimethoxysilane; and preferably, the silanide comprises at least one of methyl orthosilicate, ethyl orthosilicate, propyl orthosilicate and butyl orthosilicate.

5. The preparation method for a magnetic bead according to claim 1, **characterised in that** the preparation method for a magnetic bead further comprises: treating the surface of the magnetic core with a hydrophilic substance prior to the silanide-based modification, wherein the hydrophilic substance is any one or more of citric acid-based, acetate-based, polyethylene glycol-based and polyvinylpyrrolidone-based hydrophilic substances.

6. The preparation method for a magnetic bead according to claim 1, **characterised in that** the magnetic core is prepared in the presence of an alkaline pH regulator, and the silanide modification reaction is performed in the presence of an alkaline pH regulator.

7. The preparation method for a magnetic bead according to claim 1, **characterised in that** the magnetic core is prepared using a chemical coprecipitation method, a solvothermal method, a microemulsion method, a direct-current arc plasma method or a pyrolysis method.

8. A magnetic bead prepared by the preparation method for a magnetic bead according to any one of claims 1-7.

9. Use of the magnetic bead according to claim 8 in nucleic acid extraction.

10. The use of the magnetic bead according to claim 9, **characterised in that** the nucleic acid is adsorbed to the magnetic bead in a solution system that facilitates the adsorption of a nucleic acid to the magnetic bead, and the nucleic acid is eluted from the magnetic bead.

11. A method for extracting a nucleic acid, **characterised in that** the method for extracting a nucleic acid comprises:
providing a biological sample containing a free nucleic acid and non-nucleic acid impurities, an impurity-removing magnetic bead and a binding magnetic bead, wherein the binding magnetic bead is the magnetic bead according to claim 8, and the impurity-removing magnetic bead and the binding magnetic bead are the same or different magnetic bead(s), the differences of the two magnetic beads include that the modification groups on the surface of the two magnetic beads and/or the modification amount of the modification group on the surface of the two magnetic beads are/is different;
adsorbing the non-nucleic acid impurities to the impurity-removing magnetic bead in a solution system that does not facilitate the adsorption of the nucleic acid to the modification group, and removing the impurity-removing magnetic bead; and
adsorbing the nucleic acid to the binding magnetic bead in a solution system that facilitates the adsorption of the nucleic acid to the modification group, and eluting and harvesting the nucleic acid from the binding magnetic bead.

12. The method according to claim 11, **characterised in that** the modification group of the impurity-removing magnetic bead is silicon hydroxyl, carboxyl or a substance having ion exchange properties.

13. The method according to claim 12, **characterised in that** the modification group of the impurity-removing magnetic bead is selected from at least one of silicon hydroxyl, carboxyl and amino; the silicon hydroxyl is obtained by silanide modification; preferably, the silanide comprises at least one of methyl orthosilicate, ethyl orthosilicate, propyl orthosilicate, butyl orthosilicate, alkyltrimethoxysilane, alkyltriethoxysilane, alkyltriisopropylsilane, dialkyldimethoxysilane, dialkyldiethoxysilane, phenyltrimethoxysilane, phenyltriethoxysilane, aminopropyltrimethoxysilane and epoxypropyltrimethoxysilane, and more preferably, the silanide comprises at least one of methyl orthosilicate, ethyl orthosilicate, propyl orthosilicate and butyl orthosilicate.

14. The method according to any one of claims 11-13, **characterised in that** a sufficient amount of an alcohol and/or a chaotropic salt is not present in the solution system that does not facilitate the adsorption of the nucleic acid to the modification group; and
a sufficient amount of an alcohol and/or a chaotropic salt is present in the solution system that facilitates the adsorption of the nucleic acid to the modification group.

15. The method according to claim 14, **characterised in that** the alcohol comprises one or more of isopropyl alcohol, propanol, butanol, methanol and absolute ethanol.

16. The method according to claim 14, **characterised in that** the chaotropic salt comprises one or more of guanidine isothiocyanate, guanidine isocyanate, guanidine thiocyanate, guanidine hydrochloride, sodium iodide, lithium acetate, lithium chloride, lithium perchlorate and sodium perchlorate.

17. The method according to any one of claims 11-16, **characterised in that** the nucleic acid comprises at least one of plasmid DNA, genomic DNA, mitochondrial DNA, chloroplast DNA, a DNA fragment, total RNA, pre-mRNA, mRNA, rRNA, tRNA, ncRNA, snRNA, lncRNA and miRNA.

18. The method according to any one of claims 11-16, **characterised in that** the biological sample is from an animal, a plant or a microorganism.

19. The method according to any one of claims 11-16, **characterised in that** the biological sample is a tissue lysate or a cell lysate.

20. A nucleic acid extraction kit, **characterised in that** the nucleic acid extraction kit comprises the magnetic bead according to claim 8.

21. The kit according to claim 20, **characterised in that** the kit further comprises an impurity-removing magnetic bead, wherein the modification group of the impurity-removing magnetic bead is silicon hydroxyl, carboxyl or a substance having ion exchange properties.

22. The kit according to claim 20 or 21, **characterised in that** the modification group of the impurity-removing magnetic bead is selected from at least one of silicon hydroxyl, carboxyl and amino; the silicon hydroxyl is obtained by silanide modification; preferably, the silanide comprises at least one of methyl orthosilicate, ethyl orthosilicate, propyl orthosilicate, butyl orthosilicate, alkyltrimethoxysilane, alkyltriethoxysilane, alkyltriisopropylsilane, dialkyldimethoxysilane, dialkyldiethoxysilane, phenyltrimethoxysilane, phenyltriethoxysilane, aminopropyltrimethoxysilane and epoxypropyltrimethoxysilane, and more preferably, the silanide comprises at least one of methyl orthosilicate, ethyl orthosilicate, propyl orthosilicate and butyl orthosilicate.

23. The kit according to any one of claims 20-22, **characterised in that** the kit further comprises one or more of the following ingredients:
lysis buffer: for lysing a biological sample and releasing a nucleic acid contained therein;
neutralizing solution: for terminating the lysis reaction of the lysis buffer;
binding buffer: comprising an alcohol and/or a chaotropic salt that facilitates the adsorption of the nucleic acid to the binding magnetic bead;
wash buffer: for washing away impurities other than the nucleic acid on the binding magnetic bead;
elution buffer: for eluting the nucleic acid from the binding magnetic bead.

24. A method for extracting a nucleic acid, **characterised in that** the method for extracting a nucleic acid comprises:
providing a biological sample containing a free nucleic acid and non-nucleic acid impurities; adsorbing the non-nucleic acid impurities to an impurity-removing magnetic bead; and
removing the impurity-removing magnetic bead by adding a magnetic field to obtain a solution comprising the nucleic acid,
wherein the impurity-removing magnetic bead comprises silicon hydroxyl, carboxyl or a modification group having ion exchange properties.

25. The method according to claim 24, **characterised in that** the modification group of the impurity-removing magnetic bead is selected from at least one of silicon hydroxyl, carboxyl and amino; and preferably, the modification group is silicon hydroxyl.

26. The method according to claim 25, **characterised in that** the silicon hydroxyl is obtained by silanide modification; preferably, the silanide comprises at least one of methyl orthosilicate, ethyl orthosilicate, propyl orthosilicate, butyl orthosilicate, alkyltrimethoxysilane, alkyltriethoxysilane, alkyltriisopropylsilane, dialkyldimethoxysilane, dialkyldiethoxysilane, phenyltrimethoxysilane, phenyltriethoxysilane, aminopropyltrimethoxysilane and epoxypropyltrimethoxysilane; and more preferably, the silanide comprises at least one of methyl orthosilicate, ethyl orthosilicate, propyl orthosilicate and butyl orthosilicate.

27. The method according to any one of claims 24-26, **characterised in that** the method further comprises:
adding a binding magnetic bead to the solution comprising the nucleic acid, and adsorbing the nucleic acid in the solution system that facilitates the adsorption of the nucleic acid to the binding magnetic bead; and
eluting and harvesting the nucleic acid from the binding magnetic bead,
wherein the binding magnetic bead is selected from a magnetic bead comprising at least one of a silicon hydroxyl modification group, a carboxyl modification group and an amino modification group; and preferably, the binding magnetic bead is a magnetic bead comprising a silicon hydroxyl modification group.

28. The method according to claim 27, **characterised in that** the binding magnetic bead is obtained by performing silanide modification on the surface of a magnetic core prepared by means of a water-soluble salt of Fe²⁺ and Fe³⁺ , wherein the molar ratio of the silanide to the total amount of Fe²⁺ and Fe³⁺ is (0.5 : 1)-(4.0 : 1), and the particle size range of the magnetic bead is 0.1 µm-50 µm.

29. The method according to claim 28, **characterised in that** the molar ratio of the silanide to the total amount of Fe²⁺ and Fe³⁺ is (0.7 : 1)-(4.0 : 1), preferably (0.92 : 1)-(2.3 : 1).

30. The method according to any one of claims 24-29, **characterised in that** the biological sample is a tissue lysate or a cell lysate.
